# EUROPEAN PATENT SPECIFICATION

(11) **EP 1 494 698 B1**
(45) Date of publication and mention of the grant of the patent: **06.11.2013**
(21) Application number: 03744970.9
(22) Date of filing: 26.03.2003
(51) Int. Cl.: A61K 38/55, A61K 31/00, A61P 17/16, A61P 19/00, A61P 25/00, A61P 29/00, A61P 35/00

(54) **COMPOSITIONS AND METHODS FOR TREATING AND PREVENTING NECROSIS**
ZUSAMMENSETZUNGEN UND VERFAHREN ZUR BEHANDLUNG UND PRÄVENTION VON NEKROSE
COMPOSITIONS ET PROCEDES PERMETTANT DE TRAITER ET DE PREVENIR LA NECROSE

(30) Priority: 26.03.2002 IL 14892402
(43) Date of publication of application: 12.01.2005
(73) Proprietor: Nathan, Ilana (Helena), 84965 Omer (IL)
(72) Inventor: NATHAN, Ilana, 84965 Omer (IL); LICHTENSTEIN, Alexandra, 84508 Beer Sheva (IL)
(74) Representative: Modiano, Micaela Nadia
(86) International application number: PCT/IL2003/000253
(87) International publication number: WO 2003/079969

(56) References cited:
- EP-A2- 0 494 071
- WO-A-92/22309
- WO-A-02/096384
- JP-A- 5 078 298
- US-A- 4 683 241
- US-A- 5 216 022
- US-A- 6 150 334
- US-A- 6 159 938
- US-A1- 2001 012 524
- GROUTAS W C: "INHIBITORS OF LEUKOCYTE ELASTASE AND LEUKOCYTE CATHEPSIN G. AGENTS FOR THE TREATMENT OF EMPHYSEMA AND RELATED AILMENTS" MEDICINAL RESEARCH REVIEWS, NEW YORK, NY, US, vol. 7, no. 2, 1987, pages 227-241, XP000650561 ISSN: 0198-6325
- FOLKESSON H G: "CE-1037 Cortech", CURRENT OPINION IN ANTI-FLAMMATORY AND IMMUNOMODULATORYINVESTIGATIONAL DRUGS, CURRENT DRUGS, LONDON, vol. 2, no. 2, 1 January 2000 (2000-01-01) , pages 133-141, XP009146037, ISSN: 1464-8474
- FADOK V A ET AL: "Differential effects of apoptotic versus lysed cells on macrophage production of cytokines: Role of proteases", JOURNAL OF IMMUNOLOGY 20010501 US, vol. 166, no. 11, 1 May 2001 (2001-05-01), pages 6847-6854, ISSN: 0022-1767
- PATTON L M: "IN VIVO EVALUATION OF MDL 201,404YA, A NOVEL INHIBITOR OF HUMAN NEUTROPHIL ELASTASE", AGENTS AND ACTIONS SUPPLEMENTS, BIRKHAEUSER VERLAG, BASEL, CH, vol. 93, no. 42, 1 January 1993 (1993-01-01), pages 83-96, XP000650865, ISSN: 0379-0363
- HERBERT J M ET AL: "Biochemical and pharmacological activities of SR 26831, a potent and selective elastase inhibitor.", THE JOURNAL OF PHARMACOLOGY AND EXPERIMENTAL THERAPEUTICS FEB 1992, vol. 260, no. 2, February 1992 (1992-02), pages 809-816, ISSN: 0022-3565
- MACDONALD S J ET AL: "The discovery of a potent, intracellular, orally bioavailable, long duration inhibitor of human neutrophil elastase--GW311616A a development candidate.", BIOORGANIC & MEDICINAL CHEMISTRY LETTERS 9 APR 2001, vol. 11, no. 7, 9 April 2001 (2001-04-09), pages 895-898, ISSN: 0960-894X
- NAKATANI K ET AL: "Inhibitory effect of serine protease inhibitors on neutrophil-mediated endothelial cell injury.", JOURNAL OF LEUKOCYTE BIOLOGY FEB 2001, vol. 69, no. 2, February 2001 (2001-02), pages 241-247, ISSN: 0741-5400
- MACDONALD S J ET AL: "Intracellular inhibition of human neutrophil elastase by orally active pyrrolidine-trans-lactams.", BIOORGANIC & MEDICINAL CHEMISTRY LETTERS 22 JAN 2001, vol. 11, no. 2, 22 January 2001 (2001-01-22), pages 243-246, ISSN: 0960-894X
- MACDONALD SIMON J F ET AL: "Discovery of further pyrrolidine trans-lactams as inhibitors of human neutrophil elastase (HNE) with potential as development candidates and the crystal structure of HNE complexed with an inhibitor (GW475151).", JOURNAL OF MEDICINAL CHEMISTRY 29 AUG 2002, vol. 45, no. 18, 29 August 2002 (2002-08-29), pages 3878-3890, ISSN: 0022-2623

## Description

### Field of the Invention

The present invention relates to compositions for use in a method for treating and preventing cell necrosis. More specifically, the compositions for such use prevent or treat necrosis by means of inhibiting the activity of intracellular elastase acting in the cells undergoing necrosis.

### Background of the Invention

Elastase is a serine protease that catalyses the degradation of proteins, including elastin, a major structural protein of mammalian connective tissue. The art has suggested that the inhibition of elastase may be effective in the treatment of various conditions and diseases.

For example, US 4,683,241 indicates that elastase is believed to play an important role in the etiology of inflammatory connective tissue diseases. This patent discloses a class of phenolic esters exhibiting elastase inhibitory action.

US 5,216,022 discloses the use of aromatic esters of phenylenedialkanoates as inhibitors of human neutrophil elastase (also known as leukocyte elastase), for treating numerous neutrophil elastase-mediated conditions.

J.B. Herbert et al., The Journal of Pharmacology and Experimental Therapeutics, vol. 260, no. 2 (1992), p. 808-816 discloses the biochemical and pharmacological Activities of SR 26831 as a patent and selective elastase inhibitor of human leukocyte elastase-mediated acute and chronic lung injury.

US 6,159,938 indicates that the inhibition of endogenous vascular elastase may be effective in treating pulmonary vascular disease and other related conditions.

Necrosis is the relatively uncontrolled process of cell death following perturbation to the cellular environment, resulting in cell rupture. Necrosis may be treated by the use of high pressure oxygen.

### Summary of the Invention

The present invention is directed to claim 1.

The inventors have unexpectedly found that intracellular elastase is involved in necrotic cell death, and that the inhibition of said enzyme within the affected cells may serve as an effective tool for treating and/or preventing cell necrosis and diseases associated therewith.

Disclosed herein is a method for treating and preventing necrosis of cells and diseases associated therewith, comprising inhibiting the enzymatic activity of one or more elastase enzymes within said cells.

The above-mentioned method comprises administering to a subject a therapeutically effective amount of one or more elastase inhibiting agents, wherein said agents inhibit the enzymatic activity of intracellular elastase in the cells to be treated.

The inventors have also surprisingly found the inhibition of elastase within the affected cells may shift cell necrosis at least partially, into apoptotic cell death. Thus, disclosed is a method for treating and preventing cell necrosis and diseases associated therewith, comprising:
inhibiting the enzymatic activity of elastase within said cells; and
inhibiting apoptotic cell death.

The present invention is also directed to claim 4. Thus, the abovementioned pharmaceutical compositions comprise one or more elastase inhibitors that are capable of entering the cells to be treated, in combination with one or more suitable pharmaceutically-acceptable excipients.

According to one preferred embodiment of the invention, the abovementioned pharmaceutical compositions further comprise one or more inhibitors of apoptosis.

Also disclosed herein is to the use of one or more elastase inhibitors together with one or more inhibitors of apoptosis in the preparation of a medicament for treating and/or preventing necrosis of cells and diseases associated therewith, wherein said elastase inhibitors are capable of entering said cells.

The inhibitors of elastase activity used according to the invention for treating and preventing cell necrosis, and diseases associated therewith, are all capable of entering into the target cells, such that said inhibitors exert their inhibitory actions within said cells.

Preferably, necrosis may be treated or prevented according to the present invention in cells selected from the group consisting of neuronal cells, purkinje cell, hypocampal pyramidal cells, glial cells, cells of hematopoetic origin (such as lymphocytes and macrophages), hepatocytes, thymocytes, fibroblast, myocardial cells, epithelial cells, bronchial epithelial cells, glomeruli, lung epithelial cells, keratinocytes, gastrointestinal cells, epidermal cells, bone and cartilage cells.

Preferably, the diseases associated with cell necrosis, which may be treated and/or prevented according to the present invention, are selected from the group consisting of leukemias, lymphomas, asphyxia, incarcerated hernia, diabetes mellitus, tuberculosis, endometriosis, vascular dystrophy, cold injury, iron-load complications, complications of steroid treatment, cerebrovascular damage, gangrene, pressure sores, hepatitis, hemoglobinuria, viral sepsis, burns, hyperthermia, celiac disease, compartment syndrome, necrotizing procolitis, spiral cord injury, muscular dystrophy, tyrosemia, metabolic inherited disease, mycoplasmal disease, anthrax infection, viral infections, Anderson disease, congenital mitochondrial disease, phenylketonuria, placental infarct, syphilis, aseptic necrosis, avascular necrosis, alcoholism and necrosis associated with administration and/or self-administration with, and/or exposure to, cocaine, drugs (e.g., paracetamol, antibiotics, adriamycin, NSAID, cyclosporine) chemical toxins such as carbon tetrachloride, cyanide, methanol, ethylene glycol and mustard gas, agrochemicals such organophosphats and paraquat, heavy metals (lead, mercury), other warfare organophosphats.

### Brief Description of the Drawings

Fig. 1 graphically depicts the percentage of necrotic and apopoptic cells observed following treatment with and without oligomycin and anti-Fas.
Fig. 2 is a photographic representation of gelatin substrate gel electrophoresis results for lysates of U-937 cells treated/untreated with oligomycin and/or anti-Fas for 3 hours.
Fig. 3 is a photographic representation of gelatin substrate gel electrophoresis results obtained for lysates of U-937 cells treated/untreated with 0.5 mM KCN for 3 hours.
Fig. 4 is a photographic representation of a gelatin substrate electrophoretic gel, demonstrating that treatment of a cell lysate with KCN caused the appearance of a band of protease activity (lane B). This band disappeared when KCN was administered in the presence of 200 µM elastase inhibitor (lane C).
Fig. 5 presents results demonstrating the effect of elastase inhibitor III on KCN-induced necrosis in PC-12 cells. Panel A diagrammatically depicts the proportion of live, necrotic and apoptotic cells following various treatments. The numerical values for these proportions are given in the accompanying table. Panel B graphically depicts percentage PC-12 cell survival following treatment with KCN in the presence/absence of elastase inhibitor III.
Fig. 6 diagrammatically depicts the proportion of live, necrotic and apoptotic U-937 cells following treatment with KCN in the presence/absence of elastase inhibitor III. The numerical values for these proportions are given in the accompanying table.
Fig. 7 graphically illustrates the effects of elastase inhibitor III (panel B) and elastinal (Panel C) on Fas-induced apoptosis/necrosis in U-397 cells.
Fig. 8 graphically illustrates the percentage of necrotic and apoptotic PC-12 cells detected following treatment with/without oligomycin and/or STS.
Fig. 9 demonstrates the effect of an elastase inhibitor on STS-induced apoptosis in PC-12 cells.
Fig. 10 graphically illustrates the effect of an elastase inhibitor on STS-induced necrosis in PC-12 cells.
Fig. 11 demonstrates the effect of an elastase inhibitor on KCN-induced necrosis in PC-12 cells.
Fig. 12 graphically illustrates the effect of an elastase inhibitor on STS-induced necrosis in U-937 cells.

### Detailed Description of Preferred Embodiments

The term "necrosis", as used herein, encompasses cell necrosis states, as well as intermediates states, exhibiting necrotic and apoptotic characteristics. The term "elastase", as used herein, refers to one or more forms of said enzyme.

Compounds exhibiting elastase inhibitory profile, which are herein referred to as elastase inhibiting agents, or elastase inhibitors, are known in the art, and are disclosed, for example, by Stein et. al. [Biochemistry 25, p. 5414 (1986)], Powers et al. [Biochim. Biophys. Acta. 485, p. 156 (1977)], US 4,683,241, US 5,216,022, and US 6,159,938. Inhibitors of elastase are also commercially available from, e.g., Sigma-Aldrich or Calbiochem-Novabiochem Corporation.

Elastase inhibitors used according to the present invention are formulated together with one or more pharmaceutically acceptable carriers, which are non-toxic, inert solid, semi-solid or liquid fillers, diluent, encapsulating material or formulation auxiliary of any type. The pharmaceutical compositions can be administered to human and other mammalian subjects in any acceptable route, and preferably orally, parenterally or topically.

Solid dosage forms for oral administration include capsules, tablets, pills, powders and granules. In such solid dosage forms, the active compound is mixed with at least one inert, pharmaceutically acceptable excipient or carrier such as sodium citrate or dicalcium phosphate and/or fillers or extenders such as starches, lactose, sucrose, glucose and mannitol, binders such as carboxymethylcellulose and gelatin, humectants such as glycerol, disintegrating agents such as agar-agar, calcium carbonate and potato starch, absorbents and lubricants. The solid dosage forms can be prepared with coatings and shells according to methods known in the art.

Liquid dosage forms for oral administration include pharmaceutically acceptable solutions, emulsions, suspensions and syrups. In addition to the active compounds, the liquid dosage form may contain inert diluents commonly used in the art such as water or other solvents, solubilizing agents and emulsifiers such as ethyl alcohol, isopropyl alcohol, ethyl carbonate, ethyl acetate, propylene glycol and oils. Besides inert diluents, the oral compositions may also include adjuvants such as wetting agents, emulsifying and suspending agents, sweetening, flavoring and perfuming agents.

Injectable preparations suitable for parenteral administration are provided in the form of pharmaceutically acceptable sterile aqueous or non-aqueous solutions, dispersions, suspensions or emulsions as well as sterile powders for reconstitution into sterile injectable solutions or dispersions prior to use. Examples of suitable aqueous or non-aqueous carriers or vehicles include water, Ringer's solution and isotonic sodium chloride solution. Sterile oils may also be employed as a suitable suspending medium. The injectable formulations can be sterilized, for example, by filtration through a bacterial-retaining filter, or by incorporating sterilizing agents therein.

Dosage forms for topical or transmucosal administration of elastase inhibitors according to the invention may include pastes, creams, lotions, gels, powders, solutions and sprays. In addition to the active ingredient, the pastes creams and gels may contain excipients such as fats, oils, waxes, paraffins, starch, cellulose derivatives, polyethylene glycols, talc, zinc oxide, or mixture thereof. Powders and sprays can contain excipient such as lactose, talcs, silicic acid, aluminum hydroxide, calcium silicates and mixtures thereof.

The medical or pharmaceutical use of topical and transmucosal compositions containing elastase inhibitors (and optionally, anti-apoptotic agents) are also disclosed herein.

Other suitable formulations may be prepared by encapsulating the active ingredient in lipid vesicles or in biodegradable polymeric matrices, or by attaching said active ingredient to monoclonal antibodies. Methods to form liposomes are known in the art.

Dosage levels of active ingredients in the pharmaceutical compositions may be varied so as to obtain an amount of the elastase inhibitor that is effective to achieve the desired therapeutic response for a particular patient (i.e., a therapeutically effective amount). The selected dosage form will depend on the activity of the particular elastase inhibitor, the route of administration, the severity of the condition being treated and other factors associated with the patient being treated. Typical dose regimes are in the range of 0.1-200 mg/kg.

Also disclosed herein is the treatment or prevention of cell necrosis by means of inhibiting the enzymatic activity of intracellular elastase(s), and, in addition, inhibiting apoptotic cell death. Preferably, the inhibition of apoptotic cell death is accomplished by means of administering to subject a therapeutic effective amount of an anti-apoptotic agent, which is preferably selected from the group consisting of [R]-N-[2-heptyl]-methylpropargylamine (R-2HMP), vitamin E, vitamin D, caspase inhibitors and the hydrophilic bile salt ursodeoxycholic acid. Other methods known in the art for inhibiting apoptosis, for example, by means of regulation of expression of pro- and anti- apoptotic proteins, may also be used according to the present invention. Such methods are described, for example, by Li et al. [Acta. Anaesthesiol Sin, 38(4), p. 207-215 (2000)].

### Examples

### Experimental protocol

### 1. Models of necrosis in vitro

### Staurosporine and anti-Fas-induced necrosis

Human promonocytic U-937 cells in logarithmic phase were seeded at a concentration of 4x10⁵/ml. Afterwards the cells were washed twice and seeded again in glucose-free RPMI-1640 medium (Beit Haemek, Israel) supplemented with 2 mM pyruvate (Beit Haemek, Israel) and 10% dialyzed FCS (Gibco, BRL) for one hour.

The rat pheochromocytoma PC-12 cell line was propagated in DMEM medium (Gibco, BRL), supplemented with 5% heat-inactivated calf serum, 10% heat-inactivated horse serum, and 2 mM L-glutamine. PC-12 cells in logarithmic phase were seeded at a concentration of 1.2x 10⁵/well in 24-well plates (Cellstar). Then the cells were washed twice and maintained in glucose-free RPMI-1640 medium (Beit Haemek, Israel), and supplemented with 2 mM pyruvate and 10% dialyzed FCS for one hour. U-937 and PC-12 cells were incubated with and without 1 µM oligomycin (Sigma) for 45 min, and cells were treated with or without 1.25 µM staurosporine (STS) (Sigma) for an additional seven hours in U-937 cells or five hours in PC-12 cells. Alternatively, cells were treated with or without 100 ng/ml anti-Fas (Upstate biotechnology, USA) for the same time period.

### KCN-induced necrosis

U-937 and PC-12 cells cultured in complete RPMI-1640 medium were washed and seeded in glucose-free RPMI-1640 medium, as described above, and treated with or without 0.5 mM KCN (Merck, Germany) for seven hours with U-937 cells, or for five hours with PC-12 cells.

### 2. Testing of elastase inhibitor

200 µM elastase inhibitor III (MeOSuc-Ala-Ala-Pro-Val-CMK from Calbiochem) when added was administered 30 min before addition of the inducers. The inhibitor was dissolved in DMSO to a concentration of 100 mM. The final concentration of DMSO in the system was 0.2%, and was added to all treatments. In separate experiments, 200 µM of an elastase inhibitor (CE1037, manufactured by Cortech Inc.) was administered 30 min before addition of the inducers. The inhibitor was dissolved in PBS.

### 3. Cell death assay

### Trypan blue exclusion

At each time point, cell viability was determined by the trypan blue exclusion method (Daniel CP, Parreira A., et al. Leukemia Res. 11:191-196 (1987). Assays were performed in duplicate.

### Morphological quantification of apoptosis and necrosis

Cells undergoing morphological changes associated with apoptotic or necrotic cell death were monitored as described by McGahon et al. [Methods Cell Biol, 46: p. 153-85 (1995)]. Briefly, 1 ml of the cells was collected and centrifuged. The pellet was resuspended in a 20-fold dilution of the dye mixture (composed of 100 µg/ml acridine orange and 100 µg/ml ethidium bromide in PBS), placed on a glass slide and viewed on an inverted fluorescence microscope. A minimum of 200 cells was scored for each sample.

### Preparation of cell lysates

4x10⁷ U-937 cells, treated or untreated with the various inducers, were collected after three hours of incubation, washed twice with ice-cold PBS and resuspended at 10⁸/ml in ice-cold lysing buffer (50 nM Tris-HCl pH 7.5, 0.1 % NP-40, 1 mM DTT, 100 µM leupeptin and 100 µM TLCK). The cells were broken by the use of a polytron device (4 cycles of 7 seconds each) on ice, and the debris was pelleted by centrifugation in an ultracentrifuge at 120,000 x g for 30 minutes at 4°C. The supernatant was used for further studies or stored at -70°C. The protein content of each sample was determined by the protein assay (BioRad).

### 5. Electrophoresis

Electrophoresis on a gelatin substrate gel was performed as previously described (Distefano J. F., Cotto C. A., et al. Cancer Invest. 6, 487-498, (1988)). Proteases were reversibly inactivated by addiction of 100 µl aliquots of the cell lysates containing 200 µg protein to 50 µl of 0.625 M Tris-HCl buffer, pH 6.8, with 2.5% SDS, 10% sucrose and 0.03% phenol red. Samples were then electrophorated using 0.1% gelatin copolymerized in 11% polyacrylamide gel. After electrophoresis, the gels were subjected to three repeated immersions in 0.1 M Tris-HCl buffer, pH 7.0, containing 2.5% (V/V) Triton-x-100 in order to remove the SDS and reactivate the proteases. The gels were sliced and incubated overnight at 37° C in 0.1 M glycine-NaOH buffer, pH 7.0, with or without 100 µM TPCK (chymotrypsin-like serine protease inhibitor) and 100 µM elastinal (elastase-like serine protease inhibitor). The bands of protease activity were developed with amido black staining.

### Results

### 1. Anti-Fas-induced apoptosis/necrosis in U-937 cells

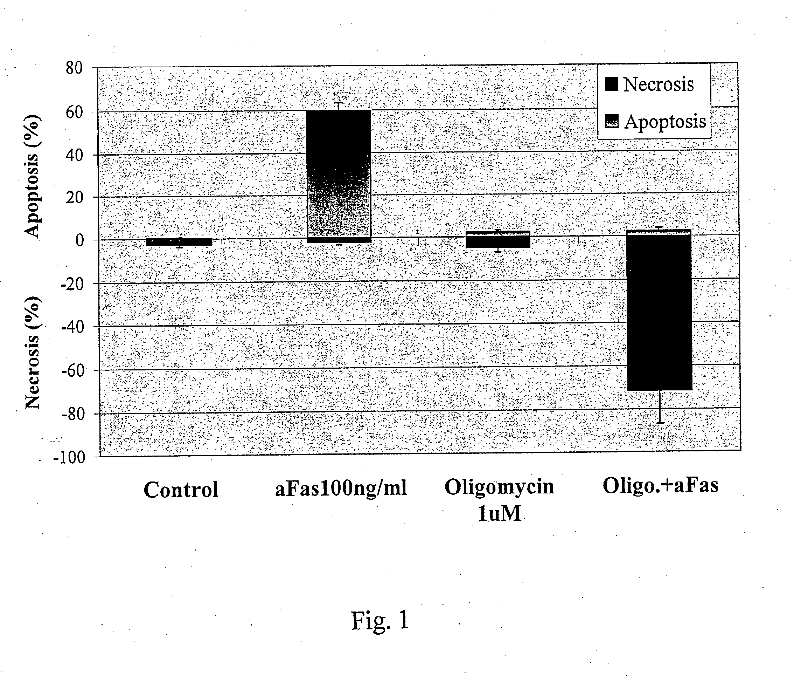

Fig. 1 indicates that treatment with anti-Fas induced about 60% apoptosis as compared to the control. Oligomycin is inactive by itself, however, addition of 100 ng/ml anti-Fas to oligomycin switched apoptotic cell death to necrotic cell death. Under these conditions, about 70% necrosis occurred and apoptosis returned to control level. Nuclear morphology was determined and analyzed by fluorescence microscope after double-staining with acridine orange and ethydium bromide.

### 2. Induction of elastase-like activity during necrotic cell death induced by anti-Fas in the presence of oligomycin

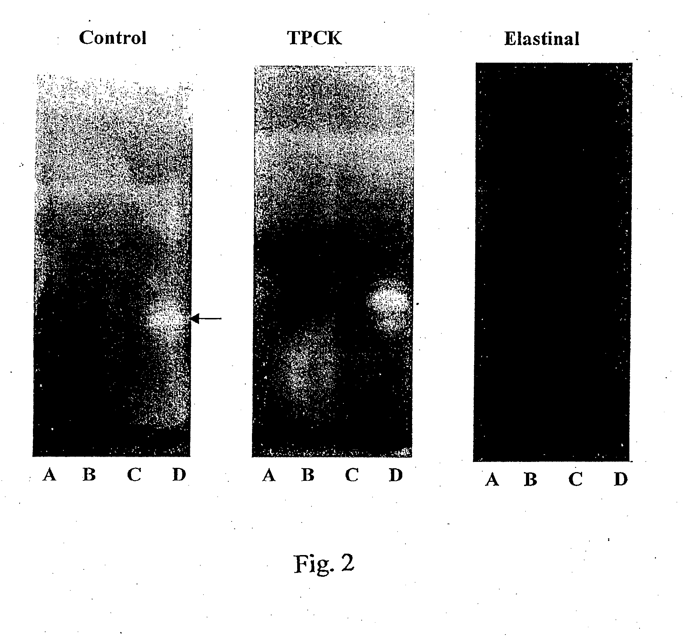

U-937 cells were maintained in glucose-free medium preincubated with or without 1 µM oligomycin for 45 min and treated with or without 100 ng/ml anti-Fas for three hours. Following this, cell lysates were prepared as described in "Experimental protocol" and applied to a gelatine substrate gel electrophoresis. The results, which are presented in Fig. 2 indicate that treatment with anti-Fas and oligomycin caused the appearance of a band of protease activity (line D), which was not found in the untreated control cells (lane A), anti-Fas-treated cells (lane B), or oligomycin-treated cells (lane C). This band disappeared in the presence of 100 µM elastinal (lane D), but not in the presence of 100 µM TPCK (lane D), indicating that treatment with anti-Fas and oligomycin induced an elastase-like activity, but not a chymotrypsin-like activity.

### 3. Induction of elastase-like activity during necrotic cell death induced by KCN

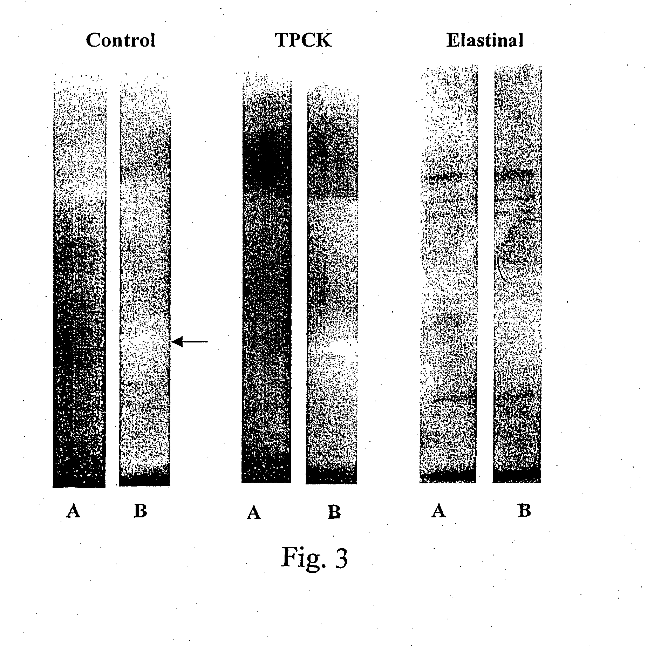

U-937 cells were treated with or without 0.5 mM KCN for three hours and then cell lysates were prepared as decribed in "Experimental protocol" and applied to a gelatine substrate gel electrophoresis. The results, which are presented in Fig. 3, show that treatment with KCN caused the appearance of a band of protease activity (lane B), which was not found in the untreated control cells (lane A). This band disappeared in the presence of 100 µM elastinal (lane B), but not in the presence of 100 µM TPCK (lane B), indicating that treatment with KCN induced an elastase-like activity, but not a chymotrypsin-like activity.

### 4. Effect of elastase inhibitor on induction of elastase-like activity during necrotic cell death

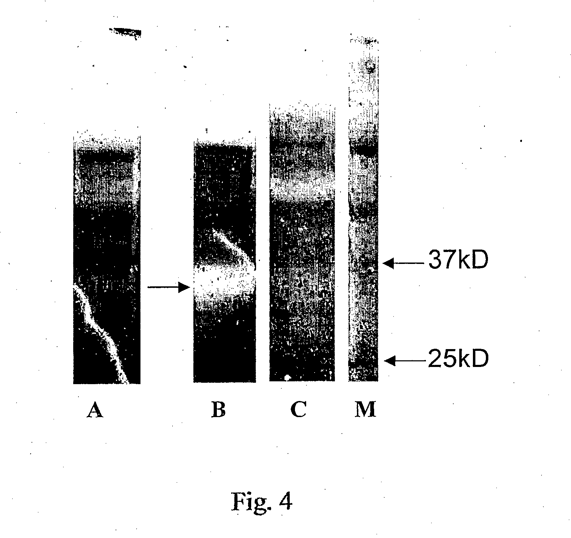

U-937 cells were treated with or without 5 mM KCN. 200 µM elastase inhibitor (Cortech) was added for three hours and then cell lysates were prepared as decribed in "Experimental protocol" and applied to a gelatine substrate gel electrophoresis. The results are presented in Fig. 4. It can be seen that treatment with KCN caused the appearance of a band of protease activity (lane B), which was not found in the untreated control cells (lane A). This band disappeared when KCN was administered in the presence of 200 µM elastase inhibitor (lane C).

### 5. Prevention of KCN-induced necrosis by elastase inhibitor III in PC-12 cells

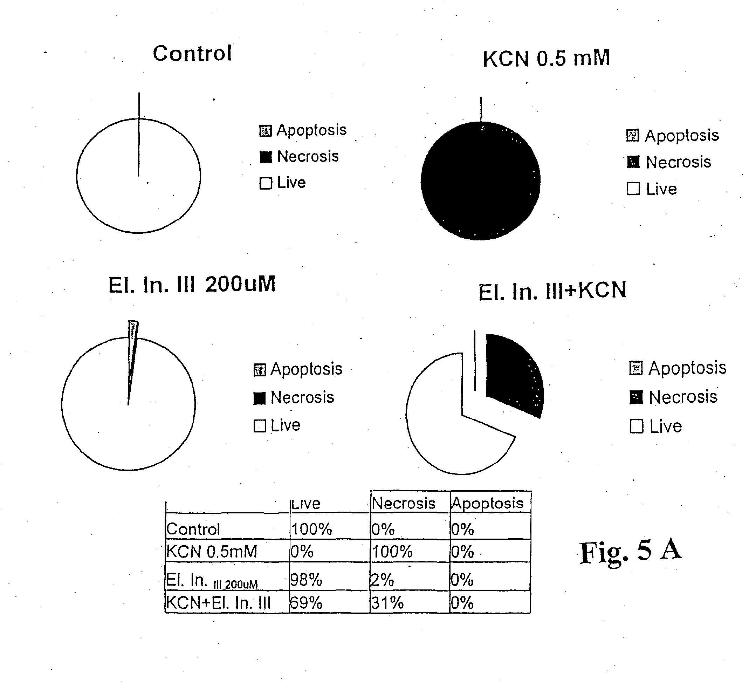

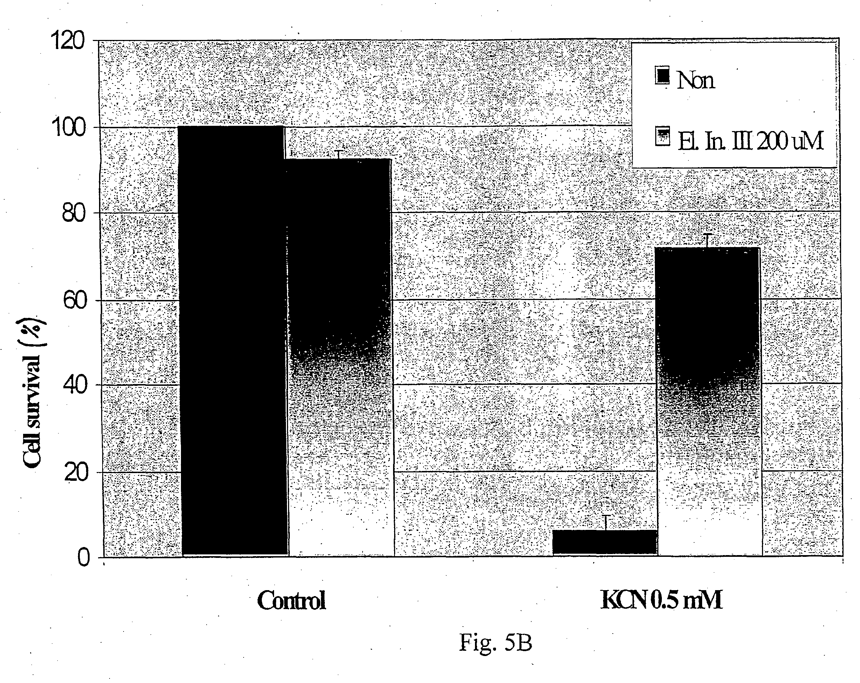

Exposure of PC-12 cells to 0.5 mM KCN induced massive necrotic cell death compared to the control. Addition of elastase inhibitor III which was inactive by itself significantly inhibited necrosis induced by KCN (Fig. 5, B). The protective effect of elastase inhibitor III is also seen when cell survival was determined under the same conditions by trypan blue exclusion (Fig. 5, A).

### 6. Inhibitory effect of elastase inhibitor III on KCN-induced necrosis in U-937 cells

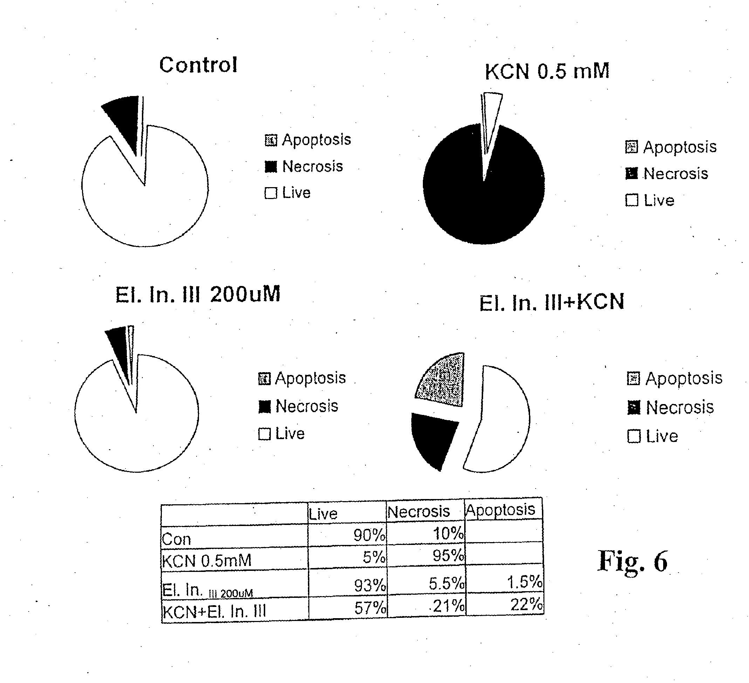

Treatment with KCN caused 95% necrosis as compared to 10% in the control. Addition of elastase inhibitor III with KCN markedly reduced necrotic cell death to 21%, and shifted 22% of the necrotic cell death to apoptotic cell death. 52% of the cells were protected from necrotic cell death by this inhibitor. Elastase inhibitor III did not cause any cell damage (Fig. 6).

### 7. Inhibitory effect of permeable versus non-permeable elastase inhibitor on anti-Fas-induced necrosis

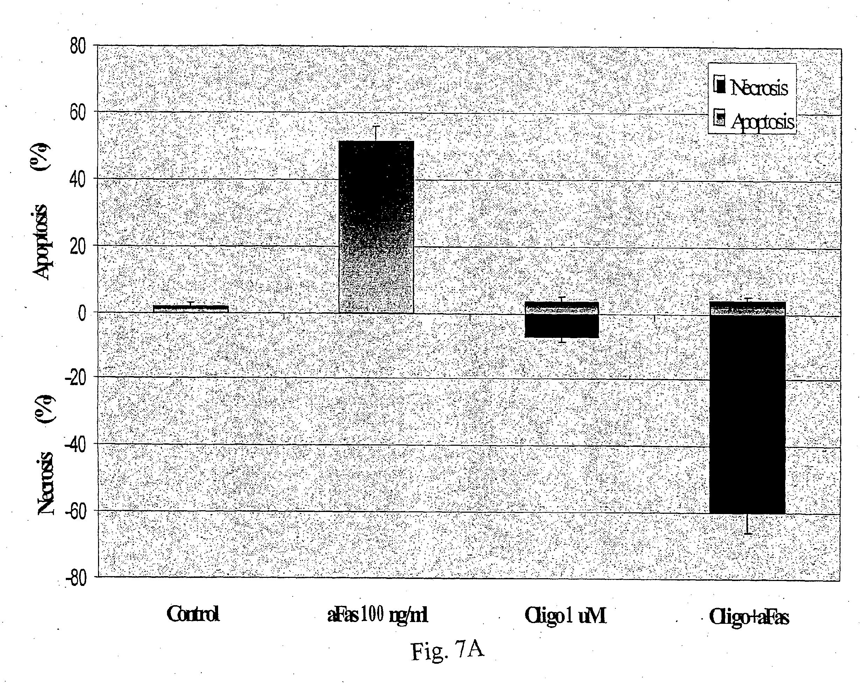

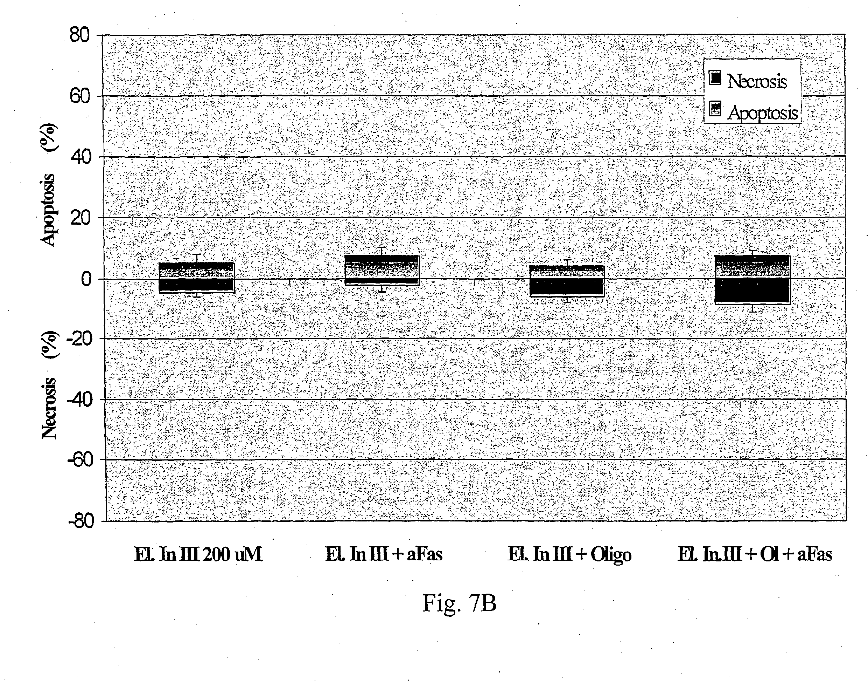

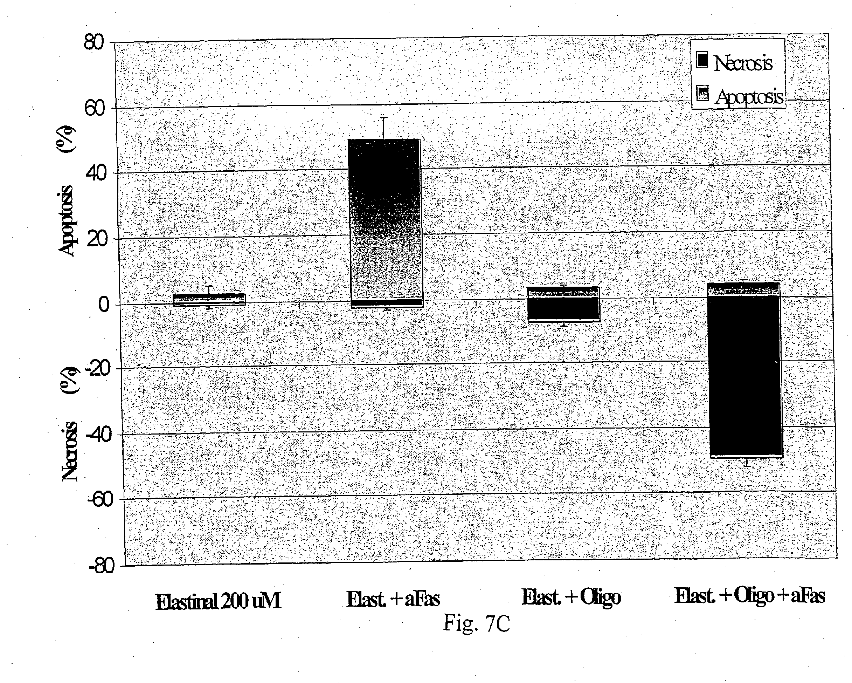

Fig. 7A shows anti-Fas-induced apoptosis/necrosis. Under these conditions cells were exposed to a permeable elastase inhibitor (Cortech Inc.). This exposure completely abrogated apoptotic as well as necrotic cell death (Fig. 7B). The non permeable elastase inhibitor-elastinal had no effect in this system (Fig. 7C).

### 8. STS-induced apoptosis/necrosis in PC-12 cells

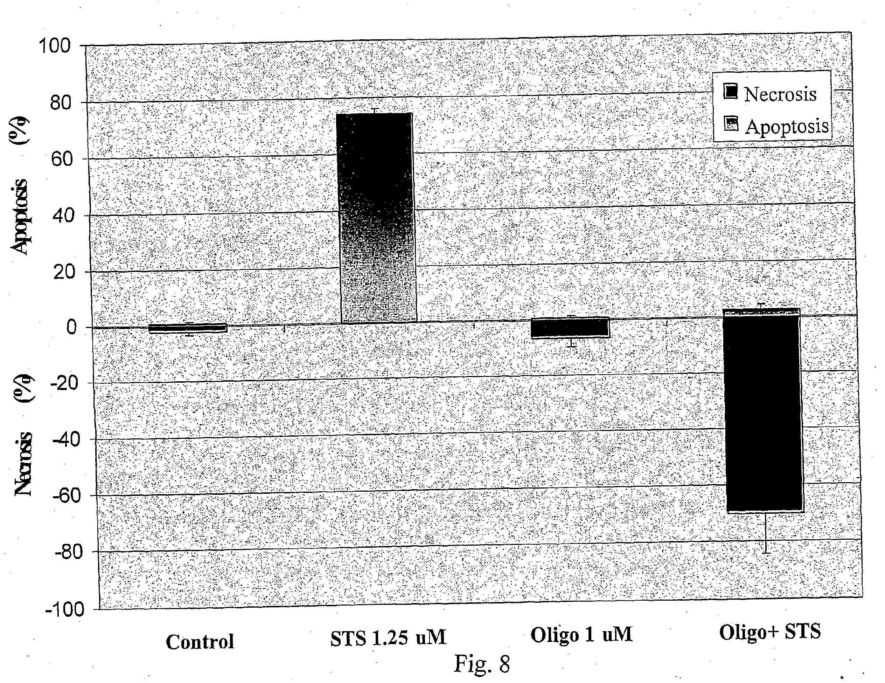

Fig. 8 indicates that treatment with 1.25 µM STS induced about 73% apoptosis as compared to the control. Oligomycin is inactive by itself, however, addition of STS to oligomycin switched apoptotic cell death to necrotic cell death. Under these conditions, about 70% necrosis occurred and apoptosis returned to control level. Nuclear morphology was determined and analyzed by fluorescence microscope after double-staining with acridine orange and ethidium bromide.

### 9. Inhibition of STS-induced apoptosis by elastase inhibitor in PC-12 cells

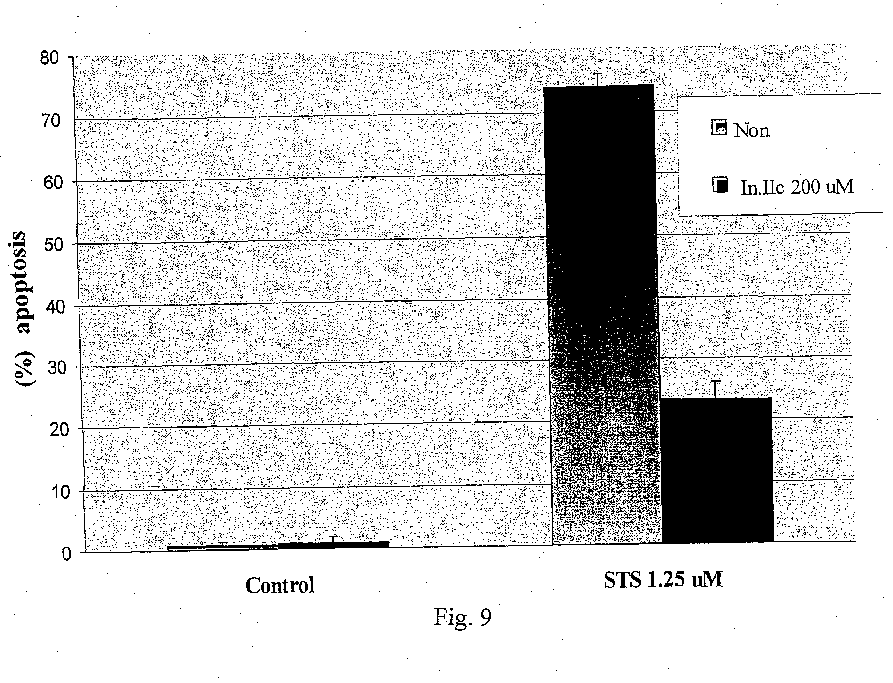

Exposure of PC-12 cells to 1.25 µM STS induced massive apoptotic cell death as compared to the control. Addition of 200 µM elastase inhibitor (Cortech, Inc.) which was inactive by itself significantly inhibited apoptosis induced by STS (Fig. 9).

### 10. Prevention of STS-induced necrosis by elastase inhibitor in PC-12 cells

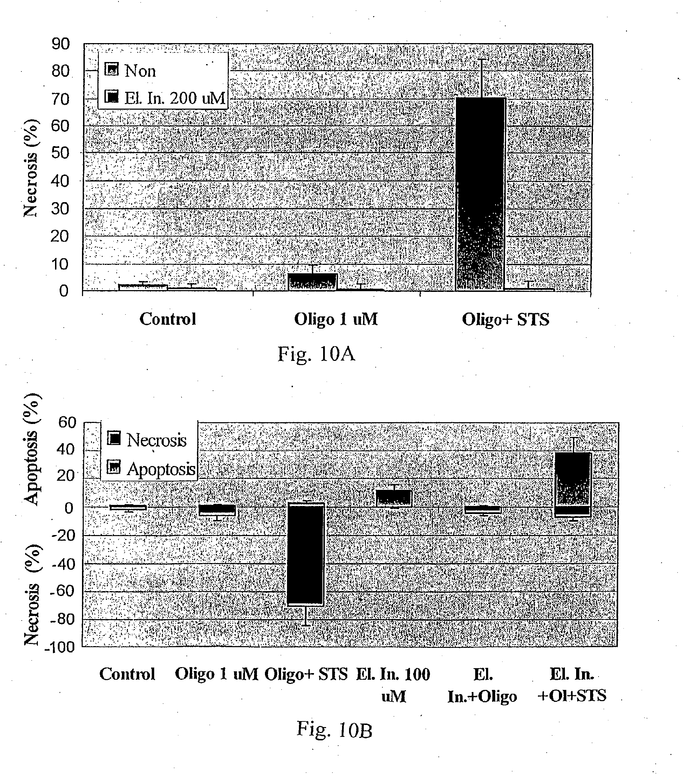

As seen in Fig. 10 A, 1.25 µM STS with 1µM oligomycin induced about 70% necrosis. 200 µM elastase inhibitor was inactive by itself, but completely abrogated necrosis-induced by STS. Under the same conditions 100 µM elastase inhibitor markedly reduced necrotic cell death to 9%, and shifted 39% of the necrotic cell death to apoptotic cell death (Fig.10B).

### 11. Inhibitory effect of elastase inhibitor on KCN-induced necrosis in PC-12 cells

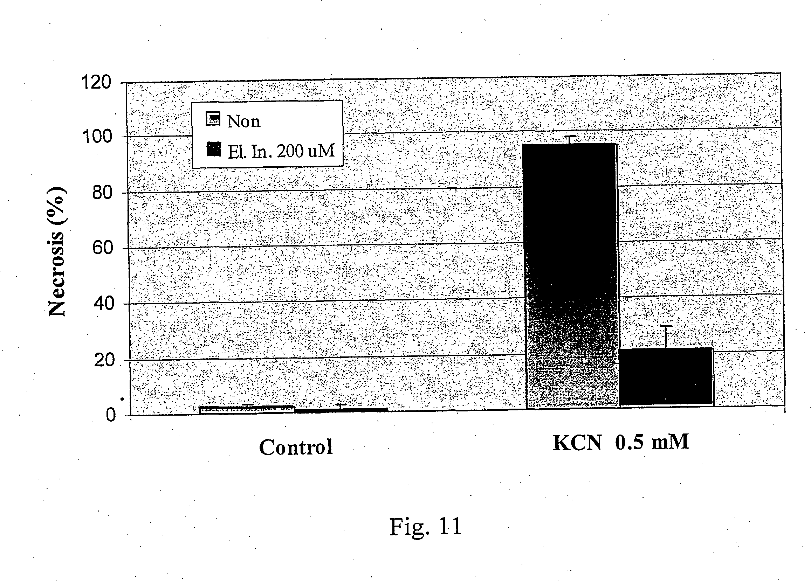

Exposure of PC-12 cells to 0.5 mM KCN induced massive necrotic cell death as compared to the control. Addition of 200 µM elastase inhibitor which was inactive by itself significantly inhibited necrosis induced by KCN (Fig. 11).

### 12. Effect of elastase inhibitor on STS-induced necrosis in U-937 cells

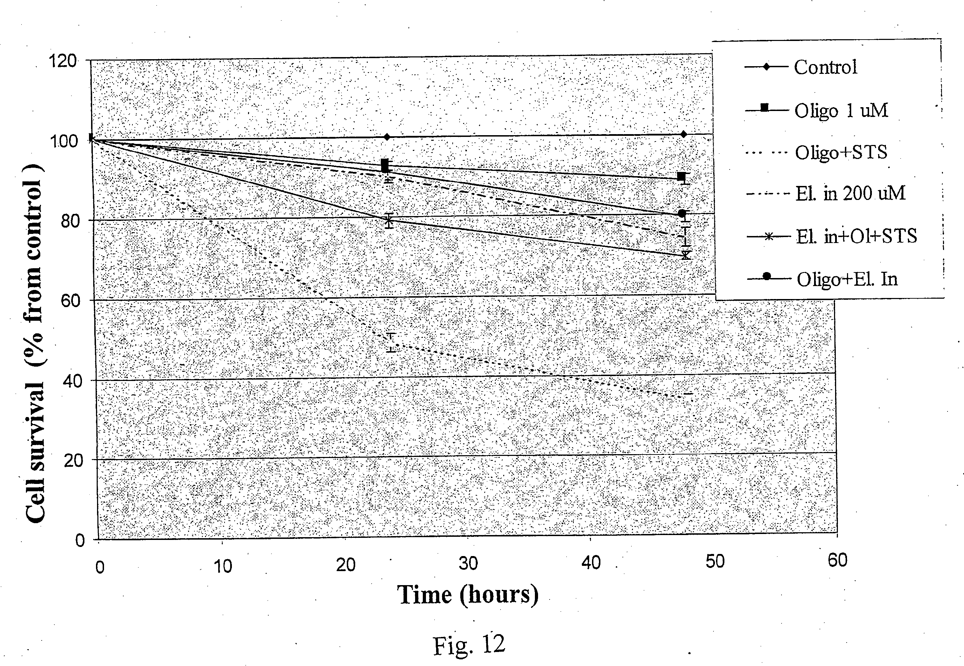

As seen in Fig. 12 treatment with STS in the presence of oligomycin markedly reduced cell survival as compared to control. Elastase inhibitor had a slight effect by itself, but it significantly inhibited cell killing induced by STS and oligomycin. The inhibitory effect was measured during prolong incubation of 48 hours. Cell viability was measured by trypan blue exclusion. Similar results were obtain for apoptosis (Data not shown).

## Claims

1. A cell permeable elastase inhibitor for use in the treatment and/or prevention of cell necrosis, wherein said elastase inhibitor inhibits the enzymatic activity of intracellular elastase involved in necrotic cell death in cells undergoing necrosis, wherein the cell necrosis is associated with a disease selected from the group consisting of necrosis associated with administration and/or self-administration with, and/or exposure to, cocaine, drugs such as paracetamol, chemical toxins, agrochemicals, heavy metals, or warfare organophosphates; vascular dystrophy; iron load complications; pressure sores; celiac disease; anthrax infection; anderson disease; congenital mitochondrial disease; aseptic necrosis; leukemia; lymphoma; asphyxia; incarcerated hernia; diabetes mellitus; tuberculosis; endometriosis; cold injury; complications of steroid treatment; cerebrovascular damage; gangrene; hepatitis; hemoglobinuria; viral sepsis; burns; hyperthermia; compartment syndrome; necrotizing procolitis; spiral cord injury; muscular dystrophy; tyrosemia; metabolic inherited disease; mycoplasmal disease; viral infections; phenylketonuria; placental infarct; syphilis; avascular necrosis; alcoholism.

2. The cell permeable elastase inhibitor for use according to claim 1, wherein said elastase inhibitor shifts necrosis at least partially into apoptotic cell death.

3. The cell permeable elastase inhibitor for use according to claim 2, wherein apoptotic cell death is to be inhibited by one or more inhibitors of apoptosis.

4. A pharmaceutical composition for use in the treatment and/or prevention of cell necrosis, wherein said composition comprises a therapeutically effective amount of a cell permeable elastase inhibitor that inhibits the enzymatic activity of one or more elastase enzymes involved in necrotic cell death in cells undergoing necrosis, and one or more pharmaceutically acceptable excipients, wherein the cell necrosis is associated with a disease selected from the group consisting of necrosis associated with administration and/or self-administration with, and/or exposure to, cocaine, drugs such as paracetamol, chemical toxins, agrochemicals, heavy metals or warfare organophosphates vascular dystrophy; iron load complications; pressure sores; celiac disease; anthrax infection; anderson disease; congenital mitochondrial disease; aseptic necrosis; leukemia; lymphoma; asphyxia; incarcerated hernia; diabetes mellitus; tuberculosis; endometriosis; cold injury; complications of steroid treatment; cerebrovascular damage; gangrene; hepatitis; hemoglobinuria; viral sepsis; burns; hyperthermia; compartment syndrome; necrotizing procolitis; spiral cord injury; muscular dystrophy; tyrosemia; metabolic inherited disease; mycoplasmal disease; viral infections; phenylketonuria; placental infarct; syphilis; avascular necrosis; alcoholism.

5. The pharmaceutical composition for the use according to claim 4, wherein said elastase inhibitor shifts necrosis at least partially into apoptotic cell death.

6. The pharmaceutical composition for the use according to claim 5, further comprising one or more inhibitors of apoptosis.

## Patentansprüche

1. Ein zellpermeabler Elastaseinhibitor zur Verwendung in der Behandlung und/oder der Prävention von Zellnekrose, worin der Elastaseinhibitor die enzymatische Aktivität von intrazellulärer Elastase hemmt, die in den nekrotischen Zelltod in Zellen, welche eine Nekrose durchlaufen, involviert ist, worin die Zellnekrose assoziiert ist mit einer Krankheit gewählt aus der Gruppe bestehend aus Nekrose assoziiert mit Verabreichung und/oder Selbstverabreichung von, und/oder dem Aussetzen gegenüber: Kokain, Arzneistoffen wie zum Beispiel Paracetamol, chemischen Toxinen, agrochemischen Mitteln, Schwermetallen, oder Kampfmittel Organophosphaten; vaskulärer Dystrophie; Eisenbelastungskomplikationen; Druckgeschwüren; Zöliakie; Milzbrandinfektion; Morbus Anderson; angeborener mitochondrialer Erkrankung; aseptischer Nekrose; Leukämie; Lymphom; Asphaxie; eingeklemmtem Leistenbruch; Diabetes Mellitus; Tuberkulose; Endometriose; Kälteschäden; Komplikationen bei der Steroidbehandlung; zerebrovaskulärer Schädigung; Wundbrand; Hepatitis; Hämoglobinurie; viraler Sepsis; Verbrennungen; Hyperthermie; Kompartmentsyndrom; nekrotisierender Procolitis; Rückenmarksverletzung; muskulärer Dystrophie; Tyrosämie; vererbter Stoffwechselkrankheit; Mykoplasmaerkrankung; viralen Infektionen; Phenylketonurie; Plazentainfarkt; Syphilis; avasukärer Nekrose; Alkoholismus.

2. Der zellpermeable Elastaseinhibitor zur Verwendung gemäß Anspruch 1, worin der Elastaseinhibitor die Nekrose zumindest teilweise in einen apoptotischen Zelltod verschiebt.

3. Der zellpermeable Elastaseinhibitor zur Verwendung gemäß Anspruch 2, worin der apoptotische Zelltod durch einen oder mehrere Inhibitoren der Apoptose verhindert werden soll.

4. Eine pharmazeutische Zusammensetzung zur Verwendung in der Behandlung und/oder der Prävention von Zellnekrose, worin die Zusammensetzung eine therapeutisch wirksame Menge eines zellpermeablen Elastaseinhibitors umfasst, der die enzymatische Aktivität von einem oder mehreren Elastaseenzymen hemmt, die in den nekrotischen Zelltod in Zellen, welche eine Nekrose durchlaufen, involviert sind, und einen oder mehrere pharmazeutisch verträgliche Bindemittel, worin die Zellnekrose mit einer Krankheit assoziiert ist, die gewählt ist aus der Gruppe bestehend aus Nekrose assoziiert mit Verabreichung und/oder Selbstverabreichung von, und/oder dem Aussetzen gegenüber: Kokain, Arzneistoffen wie zum Beispiel Paracetamol, chemischen Toxinen, agrochemischen Mitteln, Schwermetallen, oder Kampfmittel Organophosphaten; vaskulärer Dystrophie; Eisenbelastungskomplikationen; Druckgeschwüren; Zöliakie; Milzbrandinfektion; Morbus Anderson; angeborener mitochondrialer Erkrankung; aseptischer Nekrose; Leukämie; Lymphom; Asphaxie; eingeklemmtem Leistenbruch; Diabetes Mellitus; Tuberkulose; Endometriose; Kälteschäden; Komplikationen bei der Steroidbehandlung; zerebrovaskulärer Schädigung; Wundbrand; Hepatitis; Hämoglobinurie; viraler Sepsis; Verbrennungen; Hyperthermie; Kompartmentsyndrom; nekrotisierender Procolitis; Rückenmarksverletzung; muskulärer Dystrophie; Tyrosämie; vererbter Stoffwechselkrankheit; Mykoplasmaerkrankung; viralen Infektionen; Phenylketonurie; Plazentainfarkt; Syphilis; avasukärer Nekrose; Alkoholismus.

5. Die pharmazeutische Zusammensetzung zur Verwendung gemäß Anspruch 4, worin der Elastaseinhibitor die Nekrose zumindest teilweise in einen apoptotischen Zelltod verschiebt.

6. Die pharmazeutische Zusammensetzung zur Verwendung gemäß Anspruch 5, weiter einen oder mehrere Inhibitoren der Apoptose umfassend.

## Revendications

1. Inhibiteur de l'élastase perméable aux cellules destiné à être utilisé dans le traitement et/ou la prévention de la nécrose cellulaire, où ledit inhibiteur de l'élastase inhibe l'activité enzymatique de l'élastase intracellulaire impliquée dans la mort cellulaire nécrotique dans des cellules subissant une nécrose, où la nécrose cellulaire est associée à une maladie sélectionnée dans le groupe consistant en une nécrose associée à une administration et/ou à une auto-administration de, et/ou une exposition à, la cocaïne, des médicaments comme le paracétamol, des toxines chimiques, des produits agrochimiques, des métaux lourds, ou des organophosphates de guerre, la dystrophie vasculaire ; des complications associées à une charge en fer ; des escarres ; la maladie coeliaque ; une infection par l'anthrax ; la maladie d'Anderson ; une maladie mitochondriale congénitale ; la nécrose aseptique ; la leucémie ; le lymphome ; l'asphyxie ; une hernie incarcérée ; le diabète sucré ; la tuberculose ; l'endométriose ; une lésion liée au froid ; des complications d'un traitement par des stéroïdes ; une lésion cérébrovasculaire ; la gangrène ; l'hépatite ; l'hémoglobinurie ; une septicémie virale ; des brûlures ; l'hyperthermie ; le syndrome des loges ; la colite nécrosante ; une lésion de la moelle épinière ; la dystrophie musculaire ; la tyrosinémie ; une maladie héréditaire métabolique ; une maladie à mycoplasme ; des infections virales ; la phénylcétonurie ; l'infarctus placentaire ; la syphilis ; la nécrose avasculaire ; l'alcoolisme.

2. Inhibiteur de l'élastase perméable aux cellules destiné à être utilisé selon la revendication 1, où ledit inhibiteur de l'élastase modifie la nécrose au moins partiellement en mort cellulaire apoptotique.

3. Inhibiteur de l'élastase perméable aux cellules destiné à être utilisé selon la revendication 2, où la mort cellulaire apoptotique doit être inhibée par un ou plusieurs inhibiteurs de l'apoptose.

4. Composition pharmaceutique destinée à être utilisée dans le traitement et/ou la prévention de la nécrose cellulaire, où ladite composition comprend une quantité thérapeutiquement efficace d'un inhibiteur de l'élastase perméable aux cellules qui inhibe l'activité enzymatique d'une ou de plusieurs enzymes élastase impliquées dans la mort cellulaire nécrotique dans des cellules subissant une nécrose, et un ou plusieurs excipients pharmaceutiquement acceptables, où la nécrose cellulaire est associée à une maladie sélectionnée dans le groupe consistant en une nécrose associée à une administration et/ou à une auto-administration de, et/ou une exposition à, la cocaïne, des médicaments comme le paracétamol, des toxines chimiques, des produits agrochimiques, des métaux lourds, ou des organophosphates de guerre, la dystrophie vasculaire ; des complications associées à une charge en fer ; des escarres ; la maladie coeliaque ; une infection par l'anthrax ; la maladie d'Anderson ; une maladie mitochondriale congénitale ; la nécrose aseptique ; la leucémie ; le lymphome ; l'asphyxie ; une hernie incarcérée ; le diabète sucré ; la tuberculose ; l'endométriose ; une lésion liée au froid ; des complications d'un traitement par des stéroïdes ; une lésion cérébrovasculaire ; la gangrène ; l'hépatite ; l'hémoglobinurie ; une septicémie virale ; des brûlures ; l'hyperthermie ; le syndrome des loges ; la colite nécrosante ; une lésion de la moelle épinière ; la dystrophie musculaire ; la tyrosinémie ; une maladie héréditaire métabolique ; une maladie à mycoplasme ; des infections virales ; la phénylcétonurie ; l'infarctus placentaire ; la syphilis ; la nécrose avasculaire ; l'alcoolisme.

5. Composition pharmaceutique destinée à être utilisée selon la revendication 4, dans laquelle ledit inhibiteur de l'élastase modifie la nécrose au moins partiellement en mort cellulaire apoptotique.

6. Composition pharmaceutique destinée à être utilisée selon la revendication 5, comprenant en outre un ou plusieurs inhibiteurs de l'apoptose.
